# EUROPÄISCHE PATENTSCHRIFT

(11) **EP 2 415 391 B1**
(45) Veröffentlichungstag und Bekanntmachung des Hinweises auf die Patenterteilung: **15.01.2020**
(21) Anmeldenummer: 11176074.0
(22) Anmeldetag: 01.08.2011
(51) Int. Cl.: A61B 1/06, A61B 1/07, G02B 23/24, G02B 26/10, G02B 17/08, G02B 17/06

(54) **Endoskop mit einstellbarer Blickrichtung**
Endoscope with adjustable viewing direction
Endoscope à direction d'observation ajustable

(30) Priorität: 04.08.2010 DE 102010033423
(43) Veröffentlichungstag der Anmeldung: 08.02.2012
(73) Patentinhaber: Karl Storz SE & Co. KG, 78532 Tuttlingen (DE)
(72) Erfinder: Weiger, Ulrich, 78532 Tuttlingen (DE)

(56) Entgegenhaltungen:
- WO-A1-2005/003839
- WO-A1-2007/067163
- DE-T2- 60 015 375
- DE-U- 7 038 593
- US-A1- 2006 274 438
- US-A1- 2007 276 187
- US-B1- 6 364 830
- US-B1- 6 423 956

## Beschreibung

Die vorliegende Erfindung bezieht sich auf ein Endoskop mit einstellbarer Blickrichtung.

Neben Endoskopen für medizinische und nicht-medizinische technische Anwendungen, deren Blickrichtung parallel zur Längsachse des Schafts des Endoskops ist, wurden bereits früh Endoskope mit anderen feststehenden Blickrichtungen entwickelt. Mit der Blickrichtung eines Endoskops ist hier und im Folgenden immer die Richtung vom distalen Ende des Endoskops aus gemeint, in der ein Gegenstand liegt, der in der Mitte des mittels des Endoskops erfassten Bilds erscheint. Bei vielen Anwendungen ist jedoch eine feste Blickrichtung nachteilig. Im ungünstigsten Fall muss beispielsweise während eines medizinischen Eingriffs mehrfach das Endoskop gewechselt werden. In solchen Fällen ist die Verwendung eines Endoskops mit einer in situ einstellbaren bzw. verstellbaren Blickrichtung vorteilhaft.

Die Beobachtung eines Gegenstands in einem Hohlraum mittels eines Endoskops setzt in der Regel eine Beleuchtung des Gegenstands voraus. Dazu weist ein Endoskop beispielsweise Lichtwellenleiter, insbesondere Glasfasern, auf, mittels derer Beleuchtungslicht vom proximalen Ende des Endoskops entlang des Schafts zum distalen Ende des Endoskops übertragen wird. Lichtaustrittsflächen der Lichtwellenleiter am distalen Ende des Endoskops sind so angeordnet und ausgebildet, dass das Gesamtsichtfeld bzw. Blickfeld ausreichend ausgeleuchtet wird.

Bei einem Endoskop mit einstellbarer Blickrichtung wird das Beleuchtungslicht am distalen Ende des Endoskops im einfachsten Fall so verteilt, dass unabhängig von der jeweils eingestellten Blickrichtung das gesamte Sichtfeld ausgeleuchtet ist. Dies hat jedoch eine Reihe von Nachteilen zur Folge. Insbesondere wird Lichtleistung verschwendet, weil ständig unabhängig von der tatsächlich eingestellten Blickrichtung die gesamten Sichtfelder aller einstellbaren Blickrichtungen ausgeleuchtet werden. Bei einer vorbestimmten erwünschten Helligkeit muss somit insgesamt eine deutlich höhere Lichtleistung zur Verfügung gestellt werden als bei einem Endoskop mit feststehender Blickrichtung.

Ein weiterer Nachteil rührt daher, dass Beleuchtungslicht hoher Intensität Gewebe oder andere Gegenstände photothermisch oder photochemisch schädigen kann. Bei einem Endoskop mit feststehender Blickrichtung fällt ein zu geringer Abstand des distalen Endes des Endoskops zu einem Gegenstand zumindest bei Betrachtung des erfassten Bilds in der Regel auf. Bei Verwendung einer Kamera am Endoskop ist auch eine automatische Warnung von Benutzern möglich, wenn die Helligkeit eines erfassten Bilds eine vorbestimmte Schwelle überschreitet. Bei einem Endoskop mit einstellbarer Blickrichtung fällt jedoch ein Teil des Beleuchtungslichts auf Gegenstände, die außerhalb des Sichtfelds liegen. Eine unerwünschte Annäherung des distalen Endes des Endoskops an diese Gegenstände und eine resultierende Bestrahlung dieser Gegenstände mit einer zu hohen Strahlungsleistung fällt deshalb nicht auf.

Ein weiterer Nachteil besteht darin, dass auch Beleuchtungslicht, das zunächst außerhalb des Sichtfelds abgestrahlt wird, von Gegenständen oder opaken Medien gestreut oder reflektiert werden kann. Das reflektierte oder gestreute Beleuchtungslicht kann direkt oder indirekt in den Beobachtungsstrahlengang gelangen. Dadurch können Kontraste und vor allem in dunklen Bildbereichen die Unterscheidbarkeit von Gegenständen verringert werden. Weiterhin können dadurch sichtbare, störende Reflexe generiert werden.

Ein weiterer Nachteil rührt daher, dass die Beleuchtungsstärke bzw. die Intensität des Beleuchtungslichts in der Richtung, in der die Blickrichtung variiert werden kann (oft auch als vertikale Richtung bezeichnet) im Wesentlichen konstant ist, während sie in der dazu senkrechten Richtung (oft auch als horizontale Richtung bezeichnet) in der Regel zum Rand des Sichtfelds hin leicht abnimmt. Von Endoskopen mit feststehender Blickrichtung sind Benutzer jedoch in der Regel eine Beleuchtungsstärke gewohnt, die sowohl in horizontaler als auch in vertikaler Richtung zum Rand des Sichtfelds hin leicht abnimmt. Die in vertikaler Richtung konstante Beleuchtungsstärke kann deshalb als irritierend empfunden werden.

In der DE 600 15 375 T2 ist eine Anordnung mehrerer Prismen beschrieben. Eines der Prismen ist um eine Achse rotierbar, um Beleuchtungslicht in eine einstellbare Blickrichtung zu werfen. Die Erfinder der vorliegenden Erfindung haben jedoch festgestellt, dass die Verteilung des Beleuchtungslichts innerhalb des Sichtfelds mit der beschriebenen Anordnung von Prismen in vielen Fällen unbefriedigend ist. Diese ist durch die Lichtquelle oder den Faserkegel vorgegeben und kann über die Prismen nicht beeinflusst werden. In der US 6,364,830 B1 ist ein weiteres Endoskop mit einstellbarer Blickrichtung beschrieben.

In US 2006/0274438 A1 ist ein sammelndes und kondensierendes optisches System beschrieben. Das System umfasst zwei gekrümmte Spiegel, die jeweils paraboloid, ellipsoid, hyperboloid, toroidal oder sphärisch sein können. Mittels der beiden Spiegel wird Licht einer Lichtquelle, insbesondere eines Lichtbogens, einer Metall-Halogen-Lampe oder aus einer Lichtleitfaser auf ein Ziel abgebildet. Für Fall einer asymmetrischen Einhüllenden der Lichtquelle ist eine Kompensation dieses Astigmatismus mittels einer korrigierenden Optik erwähnt. Als Anwendungen sind medizinische Verfahren und insbesondere Endoskopie erwähnt. Für eine gleichmäßige Ausleuchtung ist eine Homogenisiereinrichtung erwähnt, die beispielsweise an ein Seiteverhältnis eines zu projizierenden Bildes anzupassen sei.

In WO 2007/067163 A1 ist für mikroinvasive medizinische Anwendungen eine Abtastvorrichtung zum Abtasten eines Bereichs mit einem Lichtstrahl beschrieben. Licht einer Lichtquelle wird von einem Licht leitenden Medium übertragen und von einem Abtastelement abgelenkt, um den Bereich abzutasten. Eine Abtasteinrichtung umfasst einen konvexen Spiegel, der Licht auf ein konkaves mikroelektromechanisches System lenkt.

In WO 2005/003839 A1 ist ein Verfahren zum gerichteten Bestrahlen und zur richtungsaufgelösten Detektion von Strahlung beschrieben. Ein Einkoppeleinheit zum Einkoppeln eines Lichtstrahls aus einstellbarer Richtung in einen Lichtleiter umfasst einen sphärischen Hohlspiegel.

In US 6,423,956 B1 ist eine Vorrichtung zur konfokalen Mikroskopie dargestellt, bei der ein aus einer ersten optischen Faser austretendes Licht durch eine Linse kollimiert und durch eine weitere Linse - nach Umlenkung mittels zweier Spiegel - auf ein sehr kleines konfokales Volumen gebündelt wird. Von dem konfokalen Volumen ausgehendes Licht wird - nach Umlenkung mittels zweier Spiegel - durch eine Linse kollimiert und durch eine weitere Linse auf eine zweite optische Faser gebündelt. Die Funktionen jeweils eines ebenen Spiegels und der zugeordneten Linsen können durch einen gekrümmten Spiegel erfüllt werden.

Eine Aufgabe der vorliegenden Erfindung besteht darin, ein verbessertes Endoskop mit einstellbarer Blickrichtung zu schaffen.

Diese Aufgabe wird durch den Gegenstand des unabhängigen Anspruchs gelöst. Weiterbildungen sind in den abhängigen Ansprüchen angegeben.

Ausführungsformen der vorliegenden Erfindung beruhen auf der Idee, von einer feststehenden reflektierenden Fläche und einer schwenkbaren reflektierenden Fläche zum Lenken von Beleuchtungslicht in eine einstellbare Blickrichtung mindestens eine mit einer Krümmung bzw. Wölbung zu versehen. Durch die Krümmung der feststehenden reflektierenden Fläche und/oder der schwenkbaren reflektierenden Flächen ist eine Strahlformung bzw. eine innerhalb gewisser Grenzen freie Gestaltung des Beleuchtungslicht-Bündels möglich. Einzelne Gestaltungsmöglichkeiten werden nachfolgend beschrieben.

Ein Endoskop mit einstellbarer Blickrichtung, bei dem die Blickrichtung um eine Schwenkachse schwenkbar ist, umfasst eine feststehende reflektierende Fläche zum Reflektieren von Beleuchtungslicht aus einer Richtung parallel zur Längsachse des Endoskops in eine Richtung parallel zu der Schwenkachse der Blickrichtung und eine schwenkbare reflektierende Fläche zum Reflektieren von Beleuchtungslicht aus einer Richtung parallel zur Schwenkachse der Blickrichtung in die Blickrichtung zur Beleuchtung eines mittels des Endoskops beobachteten Gegenstands, wobei zumindest entweder die feststehende reflektierende Fläche oder die schwenkbare reflektierende Fläche gekrümmt ist.

Das Beleuchtungslicht liegt an fast jeder Stelle als divergentes oder konvergentes Lichtbündel vor. Mit der Richtung des Beleuchtungslichts ist jeweils die mittlere Richtung des Beleuchtungslichts innerhalb des am betreffenden Ort vorliegenden Lichtbündels gemeint. Soweit das Beleuchtungslicht als rotationssymmetrisches Lichtbündel vorliegt, ist die Richtung des Beleuchtungslichts die Richtung der Symmetrieachse. Soweit das Beleuchtungslicht als Lichtbündel mit einem beispielsweise elliptischen Querschnitt mit zwei Symmetrieebenen vorliegt, ist die Richtung des Beleuchtungslichts die Richtung der Schnittgeraden der beiden Symmetrieebenen. Das Beleuchtungslicht wird insbesondere so geführt, dass die Richtung des Beleuchtungslichts gegebenenfalls der Richtung der optischen Achse der optischen Elemente, durch die das Beleuchtungslicht geführt wird, entspricht.

Die Längsachse des Endoskops ist insbesondere die Längsachse des Schafts. Im Fall eines starren geraden Schafts ist die Längsachse des Schafts die Gerade, auf der die Mittelpunkte der Querschnittsflächen des Schafts liegen. Im Fall eines flexiblen Schafts ist die Längsachse des Endoskops die Längsachse des distalen Endes des Schafts, also die Gerade, auf der Mittelpunkte der Querschnittsflächen des Schafts nahe dessen distalem Ende liegen. Die Schwenkachse ist insbesondere senkrecht zur Längsachse des Endoskops.

Die schwenkbare reflektierende Fläche ist insbesondere um eine Schwenkachse schwenkbar, die zu der Schwenkachse der Blickrichtung parallel oder mit dieser identisch ist.

Eine gekrümmte reflektierende Fläche kann eine kontinuierliche Krümmung aufweisen, insbesondere eine Krümmung, die entlang einer Schnittlinie mit einer Ebene konstant ist oder eine stetige oder eine glatte Funktion des Orts ist. Beispiele sind sphärische, torische (verschiedene Krümmungen bzw. Krümmungsradien in zwei zu einander senkrechten Schnittebenen), elliptische, hyperbolische Formen. Alternativ weist eine gekrümmte reflektierende Fläche beispielsweise mehreren ebenen Abschnitten auf, die unmittelbar aneinander angrenzen können.

Durch die Krümmung wirkt die feststehende reflektierende Fläche und/oder die schwenkbare reflektierende Fläche nicht einfach nur umlenkend auf das Beleuchtungslicht. Vielmehr kann eine gekrümmte reflektierende Fläche gleichzeitig eine abbildende bzw. das Lichtbündel des Beleuchtungslichts formende Wirkung haben. Durch die Integration der das Beleuchtungslicht umlenkenden und der das Beleuchtungslichtbündel formenden Wirkung in denselben beiden reflektierenden Flächen können nicht nur die Anzahl der optischen Elemente und damit auch die Herstellungskosten gering gehalten werden.

Eine Formung des Beleuchtungslicht-Bündels durch die feststehende und/oder durch die schwenkbare reflektierende Fläche ermöglicht ferner einen Eingriff genau an der Stelle, an der es konstruktiv vorteilhaft ist, wenn das Beleuchtungslicht-Bündel einen minimalen Querschnitt aufweist. Die Krümmung der feststehenden reflektierenden Fläche und/oder die Krümmung der schwenkbaren reflektierenden Fläche kann so dazu beitragen, dass eine besonders hohe Strahlungsleistung von den reflektierenden Flächen übertragen und in die einstellbare Blickrichtung geleitet werden kann.

Bei einem Endoskop, wie es hier beschrieben ist, kann die feststehende reflektierende Fläche ausgebildet und angeordnet sein, um Beleuchtungslicht zu kollimieren.

Aufgrund der kollimierenden Wirkung der feststehenden reflektierenden Fläche ist Beleuchtungslicht lichtstromabwärts der feststehenden reflektierenden Fläche insbesondere parallel oder im Wesentlichen parallel ausgerichtet.

Die parallele und insbesondere zur Schwenkachse der Blickrichtung parallele Ausrichtung des Beleuchtungslichts erleichtert eine Umlenkung des Beleuchtungslichts in eine momentan eingestellte Blickrichtung durch die schwenkbare reflektierende Fläche. Eine Fehljustierung der schwenkbaren reflektierenden Fläche kann bei Parallelität des auf die schwenkbare reflektierende Fläche fallenden Beleuchtungslichts eine geringere Auswirkung haben als bei einem konvergenten oder divergenten Lichtbündel.

Bei einem Endoskop, wie es hier beschrieben ist, kann die feststehende reflektierende Fläche die Gestalt eines Ausschnitts eines Paraboloids aufweisen.

Insbesondere weist die feststehende reflektierende Fläche die Gestalt eines Ausschnitts eines zu einer Symmetrieachse rotationssymmetrischen Paraboloids auf. Die Symmetrieachse des rotationssymmetrischen Paraboloids ist insbesondere parallel zur Schwenkachse der Blickrichtung. Insbesondere diese Ausgestaltung und Anordnung der feststehenden reflektierenden Fläche ermöglicht eine Kollimierung von Licht, das von einer am Brennpunkt des Paraboloids angeordneten Lichtaustrittsfläche ausgeht.

Im Gegensatz beispielsweise zu einer Kollimierung mittels einer Linse, die lichtstromaufwärts oder auch lichtstromabwärts einer ebenen feststehenden reflektierenden Fläche angeordnet sein könnte, ermöglicht die paraboloide feststehende reflektierende Fläche den Wegfall eines Bauteils (nämlich der Linse), einen reduzierten Justageaufwand und eine kompaktere Bauweise.

Insbesondere ist eine Lichtaustrittsfläche eines Lichtwellenleiters oder einer Lichtquelle an einem Brennpunkt des Paraboloids angeordnet.

Die Anordnung einer Lichtaustrittsfläche eines Lichtwellenleiters oder einer Lichtquelle am Brennpunkt der parabolisch geformten feststehenden reflektierenden Fläche bewirkt eine Kollimation des von der Lichtaustrittsfläche ausgehenden Beleuchtungslichts. Insbesondere bei Anordnung der Symmetrieachse des Paraboloids parallel zur Schwenkachse der Blickrichtung ist das Beleuchtungslicht somit lichtstromabwärts der reflektierenden Fläche parallel zur Schwenkachse der Blickrichtung ausgerichtet. Mittels der lichtstromabwärts angeordneten schwenkbaren reflektierenden Fläche kann das Beleuchtungslicht-Bündel weitgehend frei geformt werden. Durch die parallele Ausrichtung des Beleuchtungslichts zwischen der paraboloiden, feststehenden reflektierenden Fläche und der schwenkbaren reflektierenden Fläche kann die Ausleuchtung eines Sichtfelds weitgehend oder vollständig unabhängig davon sein, in welcher Blickrichtung das Sichtfeld liegt.

Bei einem Endoskop, wie es hier beschrieben ist, kann die schwenkbare reflektierende Fläche die Gestalt eines Ausschnitts eines Paraboloids aufweisen oder torisch sein.

Das Paraboloid ist insbesondere ein Rotationsparaboloid (d. h. rotationssymmetrisch zu einer Symmetrieachse) oder ein anderes elliptisches oder hyperbolisches Paraboloid. Eine torische Fläche weist in zwei zueinander senkrechten Schnittebenen unterschiedliche Krümmungsradien auf.

Bei einem Endoskop, wie es hier beschrieben ist, kann die Krümmung der schwenkbaren reflektierenden Fläche an das Seitenverhältnis des auszuleuchtenden Sichtfelds angepasst sein.

Insbesondere bei, wie vorstehend beschrieben, zwischen der feststehenden reflektierenden Fläche und der schwenkbaren reflektierenden Fläche kollimiertem Beleuchtungslicht kann mittels einer schwenkbaren reflektierenden Fläche mit der Gestalt eines Ausschnitts eines Rotationsparaboloids eine zur Blickrichtung rotationssymmetrische Intensität des Beleuchtungslichts lichtstromabwärts der schwenkbaren reflektierenden Fläche erzeugt werden. Dies wird von medizinischem Personal insbesondere bei unmittelbarer Beobachtung durch das Okular oder Verwendung einer Kamera, die ein Bild mit dem Seitenverhältnis 1: 1 erfasst, als vertraut und angenehm empfunden. Auch bei einem Bild mit dem Seitenverhältnis 4:3 kann eine zur Blickrichtung rotationssymmetrische Intensität des Beleuchtungslichts unter Umständen noch verwendet werden.

Es gibt jedoch einen deutlichen Trend hin zur Verwendung von HD-Kameras mit einer Auflösung von 1920 x 1200 Bildpunkten oder von 1920 x 1080 Bildpunkten und einem entsprechenden Seitenverhältnis von 16:10 oder 16:9. Bei einem solchen Seitenverhältnis kann eine zur Blickrichtung rotationssymmetrische Intensität des Beleuchtungslichts von medizinischem Personal als ungewohnt oder irritierend empfunden werden. Insbesondere bei der vorstehend beschriebenen Kollimierung des Beleuchtungslichts zwischen der feststehenden reflektierenden Fläche und der schwenkbaren reflektierenden Fläche kann die Verteilung des Beleuchtungslichts im Sichtfeld an das Seitenverhältnis des Sichtfelds angepasst werden.

Dazu weist die schwenkbare reflektierende Fläche insbesondere die Form eines nicht-rotationssymmetrischen elliptischen Paraboloids oder eines hyperbolischen Paraboloids oder eine torische Form auf, wobei die Achsen bzw. die Krümmungsradien an die Größe des Sichtfelds bzw. an dessen Seitenverhältnis angepasst ist. Mit einer Anpassung ist insbesondere gemeint, dass die ausgeleuchtete Fläche dem Sichtfeld entspricht, und/oder dass für eine (beispielsweise im Wesentlichen rechteckige oder im Wesentlichen elliptische) ebene und zur Blickrichtung senkrechte Fläche, innerhalb derer die Intensität des Beleuchtungslichts einen Mindestwert (insbesondere die Hälfte der maximalen Intensität) aufweist, das Verhältnis von Breite und Höhe dem Verhältnis von Breite und Höhe des Sichtfelds entspricht.

Bei einem Endoskop, wie es hier beschrieben ist, kann zumindest entweder die feststehende reflektierende Fläche oder die schwenkbare reflektierende Fläche an einem transparenten Körper angeordnet sein.

Ein transparenter Körper, in den Licht durch eine Lichteintrittsfläche eintritt und durch eine Lichtaustrittsfläche austritt und in dem das Licht zwischen der Lichteintrittsfläche und der Lichtaustrittsfläche an einer reflektierenden Fläche reflektiert wird, wird häufig als Prisma bezeichnet. Die reflektierende Wirkung der reflektierenden Fläche kann auf Totalreflexion oder auf einer reflektierenden Beschichtung beruhen. Bei einem Endoskop, wie es hier beschrieben ist, können die feststehende reflektierende Fläche und/oder die schwenkbare reflektierende Fläche an jeweils einem transparenten Körper angeordnet sein, deren Gestalt sich von einem Prisma im engeren Sinne der Geometrie deutlich unterscheidet. Insbesondere ist zumindest entweder die feststehende reflektierende Fläche oder die schwenkbare reflektierende Fläche gekrümmt bzw. gewölbt. Auch die Lichteintrittsfläche und die Lichtaustrittsfläche eines transparenten Körpers können jeweils konvex oder konkav gewölbt sein.

Insbesondere eine Reflexion von Licht aufgrund von Totalreflexion an einer reflektierenden Fläche des transparenten Körpers hat den Vorteil des besonders hohen, nämlich maximalen, Reflexionsgrads. Viele transparente Gläser können gut und präzise bearbeitet werden. Die Herstellung einer reflektierenden Fläche an einem transparenten Körper aus Glas kann deshalb gleichzeitig besonders gute optische Eigenschaften und eine kostengünstige Herstellung ermöglichen.

Bei einem Endoskop, wie es hier beschrieben ist, kann die feststehende reflektierende Fläche an einem transparenten Körper angeordnet sein, wobei der transparente Körper und eine Lichtaustrittsfläche eines Lichtwellenleiters oder einer Lichtquelle mittels eines transparenten Kitts gefügt sind.

Die Lichtquelle ist beispielsweise eine Leuchtdiode, ein mittels Laserlichts zur Fluoreszenz anregbarer oder auf eine andere Weise lumineszierender Körper. Die Lichtaustrittsfläche kann eine Ebene, insbesondere kreisförmige oder polygonale oder eine konkave oder konvexe Form aufweisen und dabei mehrere ebene polygonale Teilflächen umfassen. Durch Fügen der Lichtaustrittsfläche an den transparenten Körper mittels eines transparenten Kitts kann der Verlust von Strahlungsleistung des Beleuchtungslichts durch Reflexion an Grenzflächen verringert oder weitgehend oder vollständig verhindert werden. Die Lichtaustrittsfläche kann in einer Bohrung in dem transparenten Körper angeordnet sein, insbesondere am Boden bzw. Grund bzw. Ende eines Sacklochs.

Bei einem Endoskop, wie es hier beschrieben ist, können die schwenkbare reflektierende Fläche und die feststehende reflektierende Fläche so ausgebildet und angeordnet sein, dass ein am distalen Ende des Endoskops austretendes Lichtbündel von Beleuchtungslicht lichtstromabwärts der schwenkbaren reflektierenden Fläche eine Einschnürung aufweist.

Eine Einschnürung lichtstromabwärts der schwenkbaren reflektierenden Fläche kann den Platzbedarf für den Beleuchtungs-Strahlengang am distalen Ende des Endoskops reduzieren. Insbesondere kann dadurch die erforderliche Größe eines Fensterbauteils, durch das das Beleuchtungslicht am distalen Ende des Endoskops austritt, reduziert werden.

Bei einem Verfahren zum Beleuchten eines Sichtfels in einer Blickrichtung eines Endoskops, wobei die Blickrichtung des Endoskops um eine Schwenkachse schwenkbar ist, wird Beleuchtungslicht bereitgestellt und das bereitgestellte Beleuchtungslicht zuerst mittels einer feststehenden reflektierenden Fläche in eine Richtung parallel zu der Schwenkachse und dann mittels einer schwenkbaren reflektierenden Fläche in die momentan eingestellte Blickrichtung umgelenkt, wobei beim Umlenken zumindest entweder durch eine Krümmung der feststehenden reflektierenden Fläche oder durch eine Krümmung der schwenkbaren reflektierenden Fläche der von dem Beleuchtungslicht bestrahlte Raumbereich an das Sichtfeld angepasst wird.

Ein Verfahren zum Beleuchten eines Sichtfelds, wie es hier beschrieben ist, ist insbesondere mittels eines Endoskops, wie es hier beschrieben ist, ausführbar.

### Kurzbeschreibung der Figuren

Nachfolgend werden Ausführungsformen anhand der beigefügten Figuren näher erläutert. Es zeigen:
- Figur 1: eine schematische Darstellung eines Endoskops mit einstellbarer Blickrichtung;
- Figur 2: eine schematische Darstellung eines distalen Endes eines Endoskops mit einstellbarer Blickrichtung;
- Figur 3: eine schematische Darstellung eines distalen Endes eines weiteren Endoskops mit einstellbarer Blickrichtung;
- Figur 4: eine schematische Darstellung eines distalen Endes eines weiteren Endoskops mit einstellbarer Blickrichtung;
- Figur 5: eine schematische Darstellung eines distalen Endes eines weiteren Endoskops mit einstellbarer Blickrichtung;
- Figur 6: eine schematische Darstellung eines distalen Endes eines weiteren Endoskops mit einstellbarer Blickrichtung;
- Figur 7: ein schematische Flussdiagramm.

### Beschreibung von Ausführungsformen

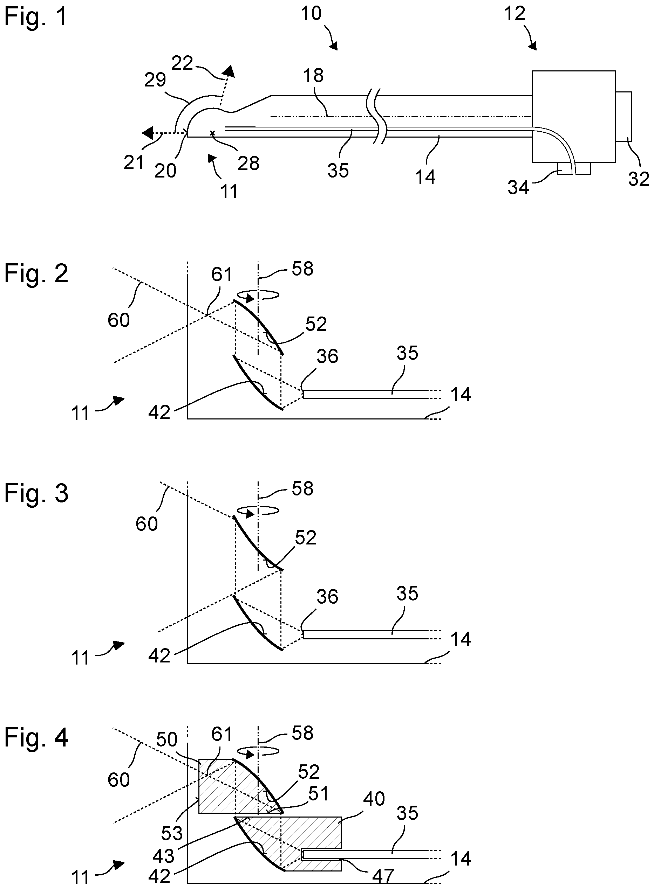

Figur 1 zeigt eine schematische Darstellung eines Endoskops 10 mit einem distalen Ende 11, einem proximalen Ende 12 und einem starren Schaft 14, der sich vom distalen Ende 11 bis zum proximalen Ende 12 erstreckt. Alternativ ist der Schaft 14 flexibel oder teilweise flexibel. Der Querschnitt des Schafts 14 oder zumindest die äußere Kontur des Querschnitts des Schafts 14 ist zwischen dem distalen Ende 11 und dem proximalen Ende 12 konstant oder im Wesentlichen konstant. Insbesondere ist die Kontur des Querschnitts des Schafts 14 kreisförmig oder elliptisch. In diesem Fall ist die in Figur 1 dargestellt Längsachse 18 des Endoskops 10 die Symmetrieachse der Mantelfläche des Schafts 14 zwischen dem distalen Ende 12 und dem proximalen Ende 11. Bei einer zylindrischen Mantelfläche des Schafts 14 ist die Längsachse 18 auch die Menge der Mittelpunkte oder Flächenschwerpunkte der Querschnitte des Schafts 14 zwischen dem distalen Ende 12 und dem proximalen Ende 11. Bei einer kreiszylindrischen Mantelfläche des Schafts 14 ist die Längsachse 18 auch die Symmetrieachse der Mantelfläche.

Am distalen Ende 12 weicht die Gestalt des Schafts 14 von der Zylindersymmetrie ab, wie dies beispielhaft in Figur 1 dargestellt ist. Insbesondere weist der Schaft 14 am distalen Ende 12 eine Öffnung auf, die durch ein transparentes Fensterbauteil mit einer gewölbten Oberfläche 20 verschlossen ist. Insbesondere verschließt das Fensterbauteil mit der gewölbten Oberfläche 20 die Öffnung hermetisch dicht. Die Oberfläche 20 des Fensterbauteils hat beispielsweise die Gestalt eines Ausschnitts eines Kreiszylindermantels, wobei die Symmetrieachse des Kreiszylinders senkrecht zur Längsachse 18 des Endoskops 10 und zur Zeichenebene der Figur 1 ist. Alternativ hat die Oberfläche 20 des transparenten Fensterbauteils die Gestalt eines Ausschnitts einer Kugeloberfläche oder eines rotationssymmetrischen oder nicht rotationssymmetrischen Ellipsoids.

Am distalen Enden 12 des Endoskops 10 sind im Schaft 14 optische Einrichtungen angeordnet, die in Figur 1 nicht dargestellt sind. Diese optischen Einrichtungen ermöglichen eine Variation der Blickrichtung des Endoskops zwischen einer ersten extremen Blickrichtung 21 und einer zweiten extremen Blickrichtung 22. Die Blickrichtung ist zwischen den beiden extremen Blickrichtungen 21, 22 um eine Schwenkachse 28 schwenkbar, die senkrecht zur Zeichenebene der Figur 1 ist. Die Blickrichtung ist jeweils die Richtung bezogen auf das distale Ende 12 des Endoskops 10, in der ein Gegenstand liegt, der in der Mitte eines mittels des Endoskops 10 erfassten Bilds erscheint.

Bei dem in Figur 1 dargestellten Beispiel ist die erste, extreme Blickrichtung 21 parallel oder im Wesentlichen parallel zur Längsachse 18 des Endoskops 10. Zwischen den extremen Blickrichtungen 21, 21 liegt ein Winkelbereich 29, der bei dem dargestellten Beispiel ca. 120 Grad umfasst. Innerhalb dieses Winkelbereichs ist die Blickrichtung des Endoskops 10 insbesondere kontinuierlich verstellbar bzw. einstellbar.

Am proximalen Ende 11 weist das Endoskop 10 eine erste Kupplung 32 zum optischen Koppeln des Endoskops 10 mit einer Kamera oder ein Okular sowie eine zweite Kupplung 34 zum Koppeln des Endoskops 10 mit einer Lichtquelle über ein Lichtleitkabel auf. Alternativ kann das Bild auch distal über einen Kamerachip aufgenommen werden. Von der zweiten Kupplung 34 führen ein oder mehrere Lichtwellenleiter 35 durch den Schaft 14 bis zum distalen Ende 11 des Endoskops 10. Von einer Lichtquelle erzeugtes Beleuchtungslicht kann über ein Lichtleitkabel, die Kupplung 34 und den oder die Lichtwellenleiter 35 zum distalen Ende 11 des Endoskops 10 übertragen werden. Einrichtungen zum Leiten des Beleuchtungslichts auf einen mittels des Endoskops 10 betrachteten Gegenstand werden anhand der Figuren 2 bis 6 dargestellt.

Die Figuren 2 bis 6 zeigen schematische Darstellungen von je einer Variante eines Endoskops 10, wie es oben anhand der Figur 1 dargestellt wurde. In den Figuren 2 bis 6 ist jeweils nur ein Ausschnitt des distalen Endes 11 des Endoskops 10 gezeigt. Dargestellt ist jeweils ein Schnitt entlang einer Ebene senkrecht zur Zeichenebene der Figur 1 und parallel zur Schwenkachse 28 der Blickrichtung 21, 22. Die dargestellten Schnittebenen schneiden insbesondere den Lichtwellenleiter 35 oder einen der Lichtwellenleiter 35. In den Figuren 2 bis 6 ist die Wand des Schafts 14 jeweils lediglich in stark vereinfachter Form als L-förmige Linie angedeutet, um eine grobe Unterscheidung zwischen dem Innenraum des Endoskops und dessen Umgebung und die Anordnung der dargestellten Elemente relativ zum distalen Ende 11 des jeweils dargestellten Endoskops 10 anzudeuten. In jeder der Figuren 2 bis 6 ist das distale Ende 11 des jeweils dargestellten Endoskops 10 in einer Konfiguration zur Beleuchtung eines Sichtfelds in der ersten extremen Blickrichtung dargestellt.

Bei jeder der in den Figuren 2 bis 6 dargestellten Ausführungsformen sind eine feststehende reflektierende Fläche 42 und eine schwenkbare reflektierende Fläche 52 vorgesehen. Bei den dargestellten Beispielen sind jeweils beide reflektierende Flächen 42, 52 gekrümmt ausgeführt. Die feststehende reflektierende Fläche 42 ist insbesondere weder translatorisch noch rotatorisch relativ zum Schaft 14 des Endoskops 10 bewegbar. Die schwenkbare reflektierende Fläche 52 ist jeweils um eine Schwenkachse 58 schwenkbar bzw. rotierbar. Die Schwenkachse 58 ist insbesondere parallel zur Schwenkachse 28 der Blickrichtung 21, 22 des Endoskops. Die Schwenkachse 58 der schwenkbaren reflektierenden Fläche 52 kann mit der Schwenkachse 28 der Blickrichtung 21, 22 identisch sein. Bei jeder der Figuren 2 bis 6 liegt die Schwenkachse 58 der schwenkbaren reflektierenden Fläche 52 in der Zeichenebene.

Alternativ sind Schwenkachse 28 der Blickrichtung 21, 22, die Schwenkachse 58 der schwenkbaren reflektierenden Fläche 52 und die schwenkbare reflektierende Fläche 52 selbst um die Längsachse 18 des Endoskops 10 oder um eine dazu parallele oder im Wesentlichen parallele Achse schwenkbar.

Figur 2 zeigt eine schematische Darstellung eines distalen Endes 11 einer Ausführungsform eines Endoskops, bei der beide reflektierenden Flächen 42, 52 konkav ausgebildet sind bzw. eine sammelnde bzw. bündelnde Eigenschaft aufweisen. Die reflektierenden Flächen 42, 52 sind beispielsweise reflektierende Oberflächen oder reflektierende Beschichtungen von Spiegeln aus Glas, Kunststoff, Keramik oder einem anderen Material. Sowohl die feststehende reflektierende Fläche 42 als auch die schwenkbare reflektierende Fläche 52 weisen jeweils die Gestalt eines Ausschnitts eines Paraboloids auf. Die feststehende reflektierende Fläche 42 weist insbesondere die Gestalt eines Ausschnitts eines Rotationsparaboloids bzw. eines rotationssymmetrischen elliptischen Paraboloids, an dessen Brennpunkt eine Lichtaustrittsfläche 36 des Lichtwellenleiters 35 angeordnet ist, auf.

Vom Lichtwellenleiter 35 übertragenes Beleuchtungslicht tritt an der Lichtaustrittsfläche 36 aus. Der Raumwinkelbereich, in dem das Beleuchtungslicht an der Lichtaustrittsfläche 36 austritt, ist aufgrund der Übertragungseigenschaften des Lichtwellenleiters 35 primär durch die Eigenschaften der Einkopplung des Beleuchtungslichts am proximalen Ende des Lichtwellenleiters 35 bestimmt. Die Einkopplung des Beleuchtungslichts am proximalen Ende des Lichtwellenleiters 35 erfolgt mittels einer transparenten Kegeleinrichtung, insbesondere eines Faserkegels oder eines Vollkegels. Mittels dieses transparenten Kegels werden beim Einkoppeln des Beleuchtungslichts am proximalen Ende des Lichtwellenleiters 35 Durchmesser und Divergenz auf für eine optimale Einkopplung geeignete Werte eingestellt. Insbesondere werden die Eigenschaften des transparenten Kegels so gewählt, dass ein möglichst großer Teil des bereitgestellten Beleuchtungslichts in den Lichtwellenleiter 35 eingekoppelt und mittels des Lichtwellenleiters 35 zum distalen Ende 11 des Endoskops 10 übertragen wird.

Das von der Lichtaustrittsfläche 36 des Lichtwellenleiters 35 ausgehende divergente Beleuchtungslicht wird von der feststehenden reflektierenden 42 Fläche kollimiert, da die Lichtaustrittsfläche 36 am Brennpunkt der parabolischen Fläche 42 angeordnet ist.

Lichtstromabwärts der feststehenden reflektierenden Fläche 42 verläuft das Beleuchtungslicht deshalb parallel bzw. im Wesentlichen parallel zur Symmetrieachse des Rotationsparaboloids. Die Symmetrieachse des Rotationsparaboloids ist parallel zur Schwenkachse 58 der schwenkbaren reflektierenden Fläche 52. Damit breitet sich das Beleuchtungslicht zwischen der feststehenden reflektierenden Fläche 42 und der schwenkbaren reflektierenden Fläche 52 parallel bzw. im Wesentlichen parallel zur Schwenkachse 58 der schwenkbaren reflektierenden Fläche 52 aus.

Das von der feststehenden reflektierenden Fläche 42 reflektierte Beleuchtungslicht wird dann von der schwenkbaren reflektierenden Fläche 52 reflektiert. Aufgrund der konkaven Krümmung der schwenkbaren reflektierenden Fläche 52 weist der von der schwenkbaren reflektierenden Fläche 52 ausgehende Beleuchtungslichtkegel eine in Figur 2 idealisiert dargestellte Einschnürung 61 auf. Der Öffnungswinkel des Beleuchtungslichtkegels 60 ist an das Sichtfeld des Endoskops angepasst. Wenn das Sichtfeld des Endoskops kreisförmig oder quadratisch ist, weist die schwenkbare reflektierende Fläche 52 beispielsweise die Gestalt eines Ausschnitts eines Rotationsparaboloids, dessen Symmetrieachse parallel zur Schwenkachse 58 der schwenkbaren reflektierenden Fläche 52 ist, auf. In diesem Fall weist der Beleuchtungslichtkegel 60 sowohl in der in Figur 2 dargestellten Schnittebene als auch in einem Schnitt entlang einer Ebene senkrecht zur Zeichenebene der Figur 2 (parallel zur Zeichenebene der Figur 1) die Einschnürung 61 am gleichen Ort auf. Der Beleuchtungslichtkegel 60 ist damit rotationssymmetrisch oder im Wesentlichen rotationssymmetrisch zu einer Symmetrieachse durch die Einschnürung 61.

Alternativ weist die schwenkbare reflektierende Fläche 52 beispielsweise die Gestalt eines nicht-rotationssymmetrischen elliptischen Paraboloids oder eine torische Gestalt auf. In diesem Fall kann der von der schwenkbaren reflektierenden Fläche 52 ausgehende Beleuchtungslichtkegel 60 in der in Figur 2 dargestellten Schnittebene und in einer dazu senkrechten Schnittebene Einschnürungen 61 an zwei verschiedenen Orten und entsprechend zwei verschiedene Öffnungswinkel aufweisen. Der Beleuchtungslichtkegel 60 kann auf diese Weise an ein Sichtfeld mit einem Seitenverhältnis, das nicht 1:1 beträgt, angepasst werden.

Die Strahlformung des Beleuchtungslichts bzw. die Gestaltung des Beleuchtungslichtkegels 60 und des von ihm ausgeleuchteten Raumwinkelbereichs sowie die Intensitätsverteilung innerhalb des Beleuchtungslichtkegels sind durch die feststehende reflektierende Fläche 42 und insbesondere durch die schwenkbare reflektierende Fläche 52 innerhalb weiter Grenzen frei einstellbar. Die Größe und die Krümmung der feststehenden reflektierenden Fläche 42 kann im Wesentlichen an eine beliebige Abstrahlcharakteristik bzw. eine beliebige Intensitätsverteilung des aus der Lichtaustrittsfläche 36 des Lichtwellenleiters 35 austretenden Beleuchtungslichts angepasst werden. Die Einkopplung des Beleuchtungslichts am proximalen Ende des Lichtwellenleiters 35 ist somit von dem auszuleuchtenden Raumwinkelbereich weitgehend oder vollständig unabhängig. Die Einkopplung des Beleuchtungslichts in den Lichtwellenleiter 35 kann deshalb - beispielsweise mittels des oben genannten transparenten Kegels - weitgehend frei an eine optimale Einkopplung bzw. an eine Übertragung einer maximalen Strahlungsleistung mittels des Lichtwellenleiters 35 angepasst werden.

Figur 3 zeigt eine schematische Darstellung eines distalen Endes 11 einer Ausführungsform eines Endoskops, die in einigen Aspekten der oben anhand der Figur 2 dargestellten Ausführungsform ähnelt. Die Ausführungsform der Figur 3 unterscheidet sich von der oben anhand der Figur 2 dargestellten Ausführungsform insbesondere dadurch, dass die schwenkbare reflektierende Fläche 52 nicht konkav, sondern konvex ausgebildet ist. Der von der schwenkbaren reflektierenden Fläche 52 ausgehende Beleuchtungslichtkegel 60 weist deshalb im Unterschied zu der oben anhand der Figur 2 dargestellten Ausführungsform keine Einschnürung 61 auf. Ähnlich wie bei der oben anhand der Figur 2 dargestellten Ausführungsform kann auch bei der Ausführungsform der Figur 3 der Beleuchtungslichtkegel 60 über die Krümmung der schwenkbaren reflektierenden Fläche 52 weitgehend an das Sichtfeld, insbesondere die Größe und das Seitenverhältnis des Sichtfelds, angepasst werden.

Auch bei der Ausführungsform der Figur 3 kann die schwenkbare reflektierende Fläche 52 die Gestalt eines Rotationsparaboloids, dessen Symmetrieachse parallel zur Schwenkachse 58 der schwenkbaren reflektierenden Fläche 52 ist, haben. Diese Gestalt der schwenkbaren reflektierenden Fläche 52 kann zur Ausleuchtung eines Sichtfelds mit einem Seitenverhältnis von 1:1 besonders geeignet sein. Durch eine andere Krümmung der schwenkbaren reflektierenden Fläche 52 kann der Beleuchtungslichtkegel 60 an ein Sichtfeld mit einem anderen Seitenverhältnis angepasst werden. Insbesondere kann die schwenkbare reflektierende Fläche 52 die Gestalt eines Ausschnitts eines nicht-rotationssymmetrischen elliptischen Paraboloids oder eines hyperbolischen Paraboloids oder eine torische Gestalt aufweisen. Zur Ausleuchtung eines Sichtfelds, dessen Seitenverhältnis von 1:1 abweicht, ist die schwenkbare reflektierende Fläche 52 insbesondere in zwei zueinander senkrechten Schnittebenen unterschiedlich stark gekrümmt.

Bei jeder der oben anhand der Figuren 2 und 3 dargestellten Ausführungsformen kann sowohl die feststehende reflektierende Fläche 42 als auch die schwenkbare reflektierende Fläche 52 jeweils abweichend von den Darstellungen in den Figuren 2 und 3 an einem transparenten Körper angeordnet sein, innerhalb dessen das Beleuchtungslicht sich teilweise ausbreitet.

Figur 4 zeigt eine schematische Darstellung des distalen Endes 11 einer Ausführungsform eines Endoskops, die in einigen Merkmalen der oben anhand der Figur 2 dargestellten Ausführungsform ähnelt. Im Gegensatz zu der oben anhand der Figur 2 dargestellten Ausführungsform sind bei der Ausführungsform der Figur 4 jedoch sowohl die feststehende reflektierende Fläche 42 als auch die schwenkbare reflektierende Fläche 52 jeweils an einem transparenten Körper 40 bzw. 50 angeordnet bzw. ausgebildet.

Das distale Ende und die in Figur 4 aus Gründen der Übersichtlichkeit nicht mit einem Bezugszeichen versehene Lichtaustrittsfläche des Lichtwellenleiters 35 sind mit einem feststehenden transparenten Körper 40 gefügt, insbesondere mittels eines transparenten Kitts. Insbesondere ist das distale Ende des Lichtwellenleiters 35 in einem Sackloch 47 im feststehenden transparenten Körper 40 angeordnet.

Die schwenkbare reflektierende Fläche 52 ist an einem schwenkbaren transparenten Körper 50 angeordnet, der mit der schwenkbaren reflektierenden Fläche 52 um die Schwenkachse 58 schwenkbar ist. Eine Lichtaustrittsfläche 43 des feststehenden transparenten Körpers 40 ist einer Lichteintrittsfläche 51 des schwenkbaren transparenten Körpers 50 gegenüber angeordnet. Die Lichtaustrittsfläche 43 des feststehenden transparenten Körpers 40 und die Lichteintrittsfläche 51 des schwenkbaren transparenten Körpers 50 sind bei dem dargestellten Beispiel jeweils eben und parallel zueinander angeordnet. Abweichend von der Darstellung in Figur 4 können sowohl die Lichtaustrittsfläche 43 des feststehenden transparenten Körpers 40 als auch die Lichteintrittsfläche 51 des schwenkbaren transparenten Körpers 50 jeweils konvex oder konkav gekrümmt sein.

Das von der schwenkbaren reflektierenden Fläche 52 des schwenkbaren transparenten Körpers 50 reflektierte Beleuchtungslicht tritt an einer Lichtaustrittsfläche 53 des schwenkbaren transparenten Körpers 50 aus diesem aus. Bei dem dargestellten Beispiel ist die Lichtaustrittsfläche 53 des schwenkbaren transparenten Körpers 50 eben. Abweichend von dem in Figur 4 dargestellten Beispiel kann die Lichtaustrittsfläche 53 des schwenkbaren transparenten Körpers 50 konvex oder konkav gekrümmt sein.

Innerhalb des feststehenden transparenten Körpers 40 und innerhalb des schwenkbaren transparenten Körpers 50 breitet sich das Beleuchtungslicht ganz ähnlich wie bei dem oben anhand der Figur 2 dargestellten Beispiel aus. Da das Beleuchtungslicht sich zwischen der feststehenden reflektierenden Fläche 42 und der schwenkbaren reflektierenden Fläche 52 parallel zur Schwenkachse 58 des schwenkbaren transparenten Körpers 50 ausbreitet, haben die Lichtaustrittsfläche 43 des feststehenden transparenten Körpers 40 und die Lichteintrittsfläche 51 des schwenkbaren transparenten Körpers 50 keine Wirkung auf die Ausbreitungsrichtung des Beleuchtungslichts, wenn sie - wie in Figur 4 angedeutet - eben und senkrecht zur Schwenkachse 58 des schwenkbaren transparenten Körpers 50 sind.

Die Brechung des Beleuchtungslichts an der Lichtaustrittsfläche 53 des schwenkbaren transparenten Körpers 50 ist in Figur 4 nicht dargestellt. Die Brechung an der Lichtaustrittsfläche 53 des schwenkbaren transparenten Körpers 50 hat beispielsweise dann keine oder nur eine geringe Auswirkung auf den Beleuchtungslichtkegel 60, wenn und soweit sie abweichend von der Darstellung in Figur 4 sphärisch mit einem Krümmungsmittelpunkt an der Einschnürung 61 ausgebildet ist.

Die Anordnung des distalen Endes des Lichtwellenleiters 35 in einer Bohrung im feststehenden transparenten Körper 40 erlaubt eine zuverlässige mechanische Verbindung. Alternativ kann die Lichtaustrittsfläche 36 des Lichtwellenleiters 35 abweichend von der Darstellung in Figur 4 stumpf an eine ebene oder gekrümmte Lichteintrittsfläche des feststehenden transparenten Körpers 40 gefügt sein.

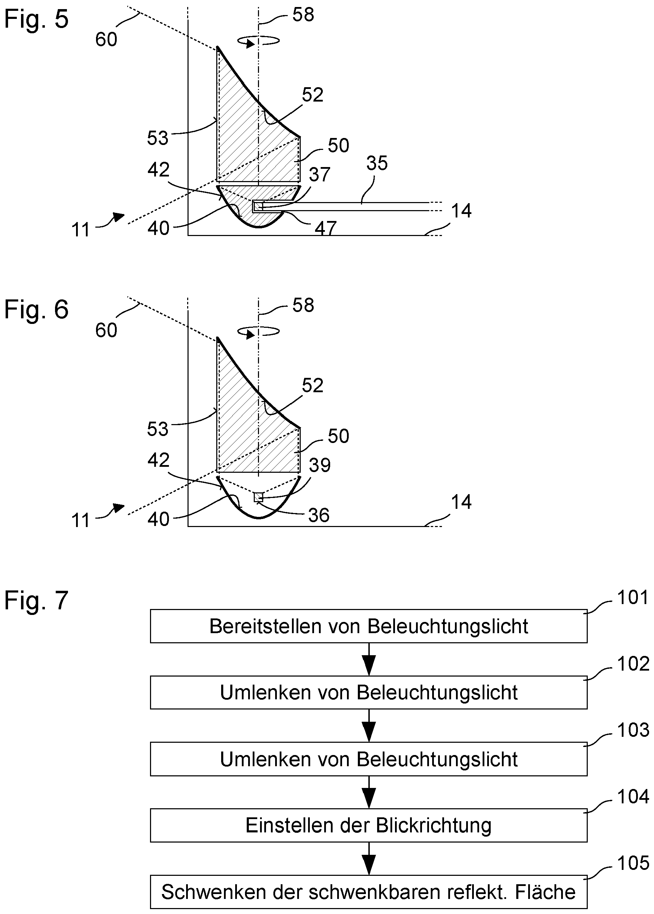

Abweichend von der Darstellung in Figur 4 kann die schwenkbare reflektierende Fläche 52 am schwenkbaren transparenten Körper 50 ähnlich wie bei der oben anhand der Figur 3 dargestellten Ausführungsform konvex zum sich im schwenkbaren transparenten Körper 50 ausbreitenden Beleuchtungslicht ausgebildet sein. Nach dem üblichen Verständnis der Kategorisierung konvex bzw. konkav einer Oberfläche eines Körpers wäre der schwenkbare transparente Körper 50 im Bereich der schwenkbaren reflektierenden Fläche 52 konkav. Beispiele mit derartig ausgebildeten schwenkbaren transparenten Körpern sind nachfolgend anhand der Figuren 5 und 6 dargestellt. Die Ausführungsformen der Figuren 5 und 6 unterscheiden sich ferner in weiteren Merkmalen von den oben anhand der Figuren 2 bis 4 dargestellten Ausführungsformen, insbesondere in der Ausgestaltung der feststehenden reflektierenden Fläche 42 und der Lichtaustrittsfläche, von der Beleuchtungslicht ausgeht.

Figur 5 zeigt eine schematische Darstellung des distalen Endes einer weiteren Ausführungsform eines Endoskops. Wie bereits erwähnt, ist der schwenkbare transparente Körper 50 im Bereich der schwenkbaren reflektierenden Fläche 52 nach innen gewölbt bzw. konkav. Bezogen auf die Ausbreitung des Beleuchtungslichts innerhalb des schwenkbaren transparenten Körpers 50 ist die schwenkbare reflektierende Fläche 52 konvex. Das im Wesentlichen parallel auf die schwenkbare reflektierende Fläche 52 treffende Beleuchtungslicht wird von der schwenkbaren reflektierenden Fläche 52 in einem divergenten Beleuchtungslichtkegel reflektiert, der keine Einschnürung aufweist. Die Wirkung der schwenkbaren reflektierenden Fläche 52 ähnelt darin der oben anhand der Figur 3 dargestellten Ausführungsform. Die Brechung des Beleuchtungslichts an der Lichtaustrittsfläche 53 des schwenkbaren transparenten Körpers 50 ist in Figur 5 nicht dargestellt.

Die Ausführungsform der Figur 5 unterscheidet sich von den oben anhand der Figuren 2 bis 4 dargestellten Ausführungsformen ferner dadurch, dass feststehende reflektierende Fläche 42 an einem rotationssymmetrischem parabolischem Teil der Oberfläche eines feststehenden transparenten Körpers 40 gebildet ist.

Die Ausführungsform der Figur 5 unterscheidet sich von den oben anhand der Figuren 2 bis 4 dargestellten Ausführungsformen ferner dadurch, dass im Brennpunkt der rotationssymmetrischen parabolischen feststehenden reflektierenden Fläche 42 ein photolumineszierender Körper 37 angeordnet ist. Der photolumineszierende Körper 37 ist insbesondere am distalen Ende des Lichtwellenleiters 35 in einem Sackloch 47 in dem feststehenden transparenten Körper 40 angeordnet. Der photolumineszierende Körper 37 weist insbesondere ein fluoreszierendes oder phosphoreszierendes Material auf, das bei Anregung mittels Anregungslichts eines Lasers oder einer anderen Lichtquelle über den Lichtwellenleiter 35 Fluoreszenz- bzw. Phosphoreszenz-Licht emittiert. Das Material des photolumineszierenden Körpers 37 ist insbesondere so gewählt, dass das Spektrum des Fluoreszenz- bzw. Phosphoreszenz-Lichts gegebenenfalls zusammen mit dem Spektrum von Anregungslicht, das vom photolumineszierenden Körper 37 gestreut wird, weiß erscheint.

Zur verlustarmen Einkopplung von mittels des Lichtwellenleiters 35 übertragenem Anregungslicht in den photolumineszierenden Körper 37 und von Fluoreszenz- bzw. Phosphoreszenz-Licht vom photolumineszierenden Körper 37 in den feststehenden transparenten Körper 40 sind der Lichtwellenleiter 35 und der photolumineszierende Körper 37 und/oder der photolumineszierende Körper 37 und der feststehende transparente Körper 40 insbesondere mittels eines transparenten Kitts mit geeignetem Brechungsindex gefügt.

Die Oberfläche des photolumineszierenden Körpers 37 stellt eine Lichtaustrittsfläche dar, von der im Wesentlichen in alle Richtungen Fluoreszenz- bzw. Phosphoreszenz-Licht ausgeht. Aufgrund der Anordnung des photolumineszierenden Körpers 37 im Brennpunkt des Rotationsparaboloids der feststehenden reflektierenden Fläche 42 breitet sich das von der feststehenden reflektierenden Fläche 42 reflektierte Beleuchtungslicht im Wesentlichen parallel zur Schwenkachse 58 des schwenkbaren transparenten Körpers 50 aus.

Figur 6 zeigt eine schematische Darstellung des distalen Endes einer weiteren Ausführungsform eines Endoskops, die in einigen Merkmalen der oben anhand der Figur 5 dargestellten Ausführungsform ähnelt. Die Ausführungsform der Figur 6 unterscheidet sich von der oben anhand der Figur 5 dargestellten Ausführungsform insbesondere darin, dass kein Lichtwellenleiter 35 vorgesehen ist, und dass anstelle eines photolumineszierenden Körpers ein lumineszierender Körper 39, beispielsweise eine organische oder anorganische Leuchtdiode, angeordnet ist. Die Oberfläche 36 des lumineszierenden Körpers 39 ist Lichtaustrittsfläche, durch die Beleuchtungslicht austritt.

Ferner unterscheidet sich die Ausführungsform der Figur 6 von der oben anhand der Figur 5 dargestellten Ausführungsform dadurch, dass kein feststehender transparenter Körper vorgesehen ist. Die feststehende reflektierende Fläche 42 ist somit keine Oberfläche eines feststehenden transparenten Körpers, sondern beispielsweise die reflektierende Oberfläche eines Spiegels. Die Ausbreitung des von dem lumineszierenden Körper 39 ausgehenden Beleuchtungslichts ähnelt der Ausbreitung des Beleuchtungslichts bei der oben anhand der Figur 5 dargestellten Ausführungsform.

Photolumineszierende oder lumineszierende Körper 37, 39 und feststehende reflektierende Flächen 42, wie sie bei den oben anhand der Figuren 5 und 6 dargestellten Ausführungsformen vorgesehen sind, können auch bei den oben anhand der Figuren 2 bis 4 dargestellten Ausführungsformen verwendet werden. Ferner sind auch bei den oben anhand der Figuren 5 und 6 dargestellten Ausführungsformen jeweils schwenkbare reflektierende Flächen 52 bzw. schwenkbare transparente Körper 50 verwendbar, wie sie oben anhand der Figuren 2 bis 4 dargestellt wurden.

Figur 7 zeigt ein schematisches Flussdiagramm eines Verfahrens zum Beleuchten eines Sichtfelds in einer Blickrichtung eines Endoskops, wobei die Blickrichtung des Endoskops um eine Schwenkachse schwenkbar ist. Obwohl das Verfahren auch mit einem Endoskop ausführbar ist, das sich von den oben anhand der Figuren 1 bis 6 dargestellten Ausführungsformen unterscheidet, werden nachfolgend beispielhaft Bezugszeichen aus den Figuren 1 bis 6 verwendet, um das Verständnis zu erleichtern.

Bei einem ersten Schritt 101 wird Beleuchtungslicht bereitgestellt. Dies erfolgt beispielsweise durch eine Leuchtdiode oder einen anderen lumineszierenden Körper 39 am distalen Ende 11 oder am proximalen Ende 12 des Endoskops 10, durch einen zur Photolumineszenz angeregten, insbesondere fluoreszierenden und/oder phosphoreszierenden Körper 37 oder durch eine andere am distalen Ende 11 oder am proximalen Ende 12 des Endoskops 10 oder außerhalb des Endoskops 10 angeordnete Lichtquelle. Gegebenenfalls wird das von der Lichtquelle erzeugte Beleuchtungslicht mittels eines Lichtwellenleiters 35 zum distalen Ende 11 des Endoskops 10 geleitet.

Bei einem zweiten Schritt 102 wird das beim ersten Schritt 101 bereitgestellte Beleuchtungslicht mittels einer feststehenden reflektierenden Fläche 42 in eine Richtung parallel zu der Schwenkachse 28 der Blickrichtung 21, 22 umgelenkt. Das Beleuchtungslicht wird dabei insbesondere aufgrund einer Krümmung der feststehenden reflektierenden Fläche 42 gleichzeitig kollimiert.

Bei einem dritten Schritt 103 wird das mittels der feststehenden reflektierenden Fläche 42 umgelenkte Beleuchtungslicht mittels einer schwenkbaren reflektierenden Fläche 52 in die momentan eingestellte Blickrichtung umgelenkt. Insbesondere wird das Beleuchtungslicht dabei nicht nur in die momentan eingestellte Blickrichtung, sondern in einen Raumwinkelbereich um die momentan eingestellte Blickrichtung, der dem Sichtfeld bei der momentan eingestellten Blickrichtung entspricht, gelenkt. Durch eine Krümmung der feststehenden reflektierenden Fläche 42 und/oder durch eine Krümmung der schwenkbaren reflektierenden Fläche 52 wird der von dem Beleuchtungslicht bestrahlte bzw. ausgeleuchtete Raumbereich bzw. Raumwinkelbereich an das Sichtfeld bei der momentan eingestellten Blickrichtung angepasst. Dies erfolgt insbesondere wie oben anhand der Figuren 2 bis 6 dargestellt.

Bei einem vierten Schritt 104 wird die Blickrichtung 21, 22 des Endoskops 10 eingestellt. Bei einem fünften Schritt 105 wird die schwenkbare reflektierende Fläche 52 entsprechend der beim vierten Schritt 104 eingestellten Blickrichtung 21, 22 geschwenkt. Der vierte Schritt 104 und der fünfte Schritt 105 werden insbesondere gleichzeitig ausgeführt. Der vierte Schritt 104 und der fünfte Schritt 105 können bereits vor dem ersten Schritt 101 ausgeführt werden.

### Bezugszeichen

- 10: Endoskop
- 11: distales Ende des Endoskops 10
- 12: proximales Ende des Endoskops 10
- 14: Schaft des Endoskops 10
- 18: Längsachse des Endoskops 10

- 20: Oberfläche eines Fensterbauteils
- 21: erste extreme Blickrichtung (0°)
- 22: zweite extreme Blickrichtung (120°)
- 28: Schwenkachse der Blickrichtung
- 29: Winkelbereich der Blickrichtungen

- 32: erste Kupplung
- 34: zweite Kupplung
- 35: Lichtwellenleiter
- 36: Lichtaustrittsfläche des Lichtwellenleiters 35 oder des lumineszierenden Körpers 39
- 37: photolumineszierender Körper am distalen Ende des Lichtwellenleiters 35
- 39: lumineszierender Körper
- 40: feststehender transparenter Körper
- 42: feststehende reflektierende Fläche des feststehenden transparenten Körpers 40
- 43: Lichtaustrittsfläche des feststehenden transparenten Körpers 40
- 47: Sackloch im feststehenden transparenten Körper 40
- 48: Schwenkachse des feststehenden transparenten Körpers 40

- 50: schwenkbarer transparenter Körper
- 51: Lichteintrittsfläche des schwenkbaren transparenten Körpers 50
- 52: schwenkbare reflektierende Fläche des schwenkbaren transparenten Körpers 50
- 53: Lichtaustrittsfläche des schwenkbaren transparenten Körpers 50
- 58: Schwenkachse des schwenkbaren transparenten Körpers 50

- 60: Beleuchtungslichtkegel bei der ersten Blickrichtung 21
- 61: Einschnürung des Beleuchtungs-Lichtkegels 60

- 101: erster Schritt
- 102: zweiter Schritt
- 103: dritter Schritt
- 104: vierter Schritt
- 105: fünfter Schritt

## Patentansprüche

1. **Endoskop** (10) **mit einstellbarer Blickrichtung** (21, 22), wobei die Blickrichtung (21, 22) um eine Schwenkachse (28) schwenkbar ist, mit Lichtwellenleitern (35), mittels derer Beleuchtungslicht vom proximalen Ende (11) des Endoskops (10) entlang des Schafts (14) zum distalen Ende (12) des Endoskops übertragen wird, mit:
einer **feststehenden reflektierenden Fläche** (42) zum Reflektieren des Beleuchtungslichts aus einer Richtung parallel zur Längsachse (18) des Endoskops (10) in eine Richtung parallel zu der Schwenkachse (28) der Blickrichtung;
einer **schwenkbaren reflektierenden Fläche** (52) zum Reflektieren des Beleuchtungslichts aus einer Richtung parallel zur Schwenkachse (28) der Blickrichtung (21, 22) in die Blickrichtung (21, 22) zur Beleuchtung eines mittels des Endoskops (10) beobachteten Gegenstands,
**dadurch gekennzeichnet, dass** zumindest entweder die feststehende reflektierende Fläche (42) oder die schwenkbare reflektierende Fläche (52) **gekrümmt** ist, um einen von Beleuchtungslicht bestrahlten Raumbereich an das Sichtfeld anzupassen.

2. Endoskop (10) nach dem vorangehenden Anspruch, bei dem die feststehende reflektierende Fläche (42) ausgebildet und angeordnet ist, um Beleuchtungslicht zu **kollimieren.**

3. Endoskop (10) nach einem der vorangehenden Ansprüche, bei dem die **feststehende** reflektierende Fläche (42) die Gestalt eines Ausschnitts eines **Paraboloids** aufweist.

4. Endoskop (10) nach dem vorangehenden Anspruch, bei dem eine **Lichtaustrittsfläche** (36) eines Lichtwellenleiters (35) oder einer Lichtquelle (39) an einem **Brennpunkt** des Paraboloids angeordnet ist.

5. Endoskop (10) nach einem der vorangehenden Ansprüche, bei dem die **schwenkbare** reflektierende Fläche (52) die Gestalt eines Ausschnitts eines **Paraboloids** aufweist oder **torisch** ist.

6. Endoskop (10) nach einem der vorangehenden Ansprüche, bei dem die **Krümmung** der schwenkbaren reflektierenden Fläche an das **Seitenverhältnis** des auszuleuchtenden Sichtfelds angepasst ist.

7. Endoskop (10) nach einem der vorangehenden Ansprüche, bei dem zumindest entweder die feststehende reflektierende Fläche (42) oder die schwenkbare reflektierende Fläche (53) an einem **transparenten Körper** (40, 50) angeordnet ist.

8. Endoskop (10) nach einem der vorangehenden Ansprüche, bei dem die feststehende reflektierende Fläche (42) an einem **transparenten Körper** (40) angeordnet ist, wobei der transparente Körper (40) und eine Lichtaustrittsfläche (36) eines Lichtwellenleiters (35) oder einer Lichtquelle (39) mittels eines transparenten Kitts gefügt sind.

9. Endoskop (10) nach einem der vorangehenden Ansprüche, bei dem die schwenkbare reflektierende Fläche (52) und die feststehende reflektierende Fläche (42) so ausgebildet und angeordnet sind, dass ein am distalen Ende des Endoskops (10) austretendes Lichtbündel (60) von Beleuchtungslicht lichtstromabwärts der schwenkbaren reflektierenden Fläche eine **Einschnürung** (61) aufweist.

## Claims

1. Endoscope (10) with an adjustable viewing direction (21, 22), wherein the viewing direction (21, 22) is pivotable about a pivot axis (28), comprising optical waveguides (35), by means of which the illumination light is transferred along the shaft (14) from the proximal end (11) of the endoscope (10) to the distal end (12) of the endoscope, comprising:
a stationary reflective surface (42) for reflecting the illumination light from a direction parallel to the longitudinal axis (18) of the endoscope (10) into a direction parallel to the pivot axis (28) of the viewing direction;
a pivotable reflective surface (52) for reflecting the illumination light from a direction parallel to the pivot axis (28) of the viewing direction (21, 22) into the viewing direction (21, 22) for illuminating an object observed by means of the endoscope (10),
**characterized in that**
at least either the stationary reflective surface (42) or the pivotable reflective surface (52) is curved in order to adapt a spatial region irradiated by the illumination light to the visual field.

2. Endoscope (10) according to the preceding claim, wherein the stationary reflective surface (42) is embodied and disposed to collimate illumination light.

3. Endoscope (10) according to either of the preceding claims, wherein the stationary reflective surface (42) has the form of a section of a paraboloid.

4. Endoscope (10) according to the preceding claim, wherein a light exit surface (36) of an optical waveguide (35) or of a light source (39) is disposed at a focus of the paraboloid.

5. Endoscope (10) according to any one of the preceding claims, wherein the pivotable reflective surface (52) has the form of a section of a paraboloid or is toric.

6. Endoscope (10) according to any one of the preceding claims, wherein the curvature of the pivotable reflective surface is adapted to the aspect ratio of the visual field to be illuminated.

7. Endoscope (10) according to any one of the preceding claims, wherein at least either the stationary reflective surface (42) or the pivotable reflective surface (53) is disposed at a transparent body (40, 50) .

8. Endoscope (10) according to any one of the preceding claims, wherein the stationary reflective surface (42) is disposed at a transparent body (40), wherein the transparent body (40) and a light exit surface (36) of optical waveguide (35) or of a light source (39) are joined by means of a transparent cement.

9. Endoscope (10) according to any one of the preceding claims, wherein the pivotable reflective surface (52) and the stationary reflective surface (42) are embodied and disposed in such a way that a light beam (60) of illumination light emerging at the distal end of the endoscope (10) has a waist (61) downstream of the pivotable reflective surface in the beam path.

## Revendications

1. Endoscope (10) doté d'une direction d'observation (21, 22) réglable, dans lequel la direction d'observation (21,22) peut pivoter autour d'un axe de pivot (28), doté de conducteurs optiques (35), au moyen desquels la lumière d'éclairage est transmise depuis une extrémité proximale (11) de l'endoscope (10) jusqu'à une extrémité distale (12) de l'endoscope le long de la tige (14), doté :
d'une surface réfléchissante fixe (42) destinée à réfléchir la lumière d'éclairage depuis une direction parallèle à l'axe longitudinal (18) de l'endoscope (10) dans une direction parallèle à l'axe de pivot (28) de la direction d'observation ;
d'une surface réfléchissante pivotante (52) destinée à réfléchir la lumière d'éclairage depuis une direction parallèle à l'axe de pivot (28) de la direction d'observation (21, 22) dans la direction d'observation (21, 22) afin d'éclairer un objet observé au moyen de l'endoscope (10),
**caractérisé en ce qu'**au moins ou bien la surface réfléchissante fixe (42) ou bien la surface réfléchissante pivotante (52) sont courbées, afin d'adapter une zone spatiale illuminée par la lumière d'éclairage au champ de vision.

2. Endoscope (10) selon l'une des revendications précédentes, où la surface réfléchissante fixe (42) est réalisée et agencée pour collimater la lumière d'éclairage.

3. Endoscope (10) selon l'une des revendications précédentes, où la surface réfléchissante fixe (42) possède la forme d'une section d'un paraboloïde.

4. Endoscope (10) selon l'une des revendications précédentes, où une surface de sortie de lumière (36) d'un conducteur optique (35) ou d'une source lumineuse (39) est agencée à un foyer du paraboloïde.

5. Endoscope (10) selon l'une des revendications précédentes, où la surface réfléchissante pivotante (52) possède la forme d'une section d'un paraboloïde ou est torique.

6. Endoscope (10) selon l'une des revendications précédentes, où la courbure de la surface réflective pivotante est adaptée au rapport largeur/hauteur du champ de vision à éclairer.

7. Endoscope (10) selon l'une des revendications précédentes, où au moins ou bien la surface réfléchissante fixe (42) ou bien la surface réfléchissante pivotante (53) est agencée à un corps transparent (40, 50).

8. Endoscope (10) selon l'une des revendications précédentes, où la surface réfléchissante fixe (42) est agencée à un corps transparent (40), dans lequel le corps transparent (40) et une surface de sortie de lumière (36) d'un conducteur optique (35) ou d'une source lumineuse (39) sont de préférence joints au moyen d'un mastic transparent.

9. Endoscope (10) selon l'une des revendications précédentes, où la surface réfléchissante pivotante (52) et la surface réfléchissante fixe (42) sont réalisées et agencées de telle sorte qu'un faisceau lumineux (60) de lumière d'éclairage émanant à l'extrémité distale de l'endoscope (10) comporte une constriction (61) en aval de la surface réfléchissante pivotante.
